# NEUE EUROPÄISCHE PATENTSCHRIFT

(11) **EP 1 894 563 B2**
(45) Veröffentlichungstag und Bekanntmachung des Hinweises auf die Entscheidung über den Einspruch: **27.04.2022**
(45) Hinweis auf die Patenterteilung: 27.10.2010
(21) Anmeldenummer: 07018343.9
(22) Anmeldetag: 20.05.2003
(51) Int. Cl.: A61K 9/70, A61K 47/32

(54) **Pflaster, enthaltend Fentanylum**
Plaster containing fentanylum
Pavé contenant du fentanyl

(30) Priorität: 28.05.2002 DE 10223835
(43) Veröffentlichungstag der Anmeldung: 05.03.2008
(62) Teilanmeldung aus: 03755897.0
(73) Patentinhaber: ratiopharm GmbH, 89079 Ulm (DE)
(72) Erfinder: Cordes, Günter, 42799 Leichlingen (DE); Vollmer, Ulrike, 41542 Dormagen (DE)
(74) Vertreter: Lederer & Keller Patentanwälte Partnerschaft mbB

(56) Entgegenhaltungen:
- EP-A- 0 622 075
- EP-A- 0 887 075
- WO-A-02/26217
- WO-A1-02/074286
- DE-C1- 10 141 650
- US-A- 5 474 783
- ROY S D ET AL: "CONTROLLED TRANSDERMAL DELIVERY OF FENTANYL: CHARACTERIZATIONS OF PRESSURE-SENSITIVE ADHESIVES FOR MATRIX PATCH DESIGN" JOURNAL OF PHARMACEUTICAL SCIENCES, AMERICAN PHARMACEUTICAL ASSOCIATION. WASHINGTON, US, Bd. 85, Nr. 5, 1. Mai 1996 (1996-05-01), Seiten 491-495, XP000583527 ISSN: 0022-3549

## Beschreibung

Die Erfindung betrifft ein transdermales therapeutisches System mit einer Deckschicht, einer Klebermatrix mit einem Gehalt an Fentanyl als Wirkstoff und mit einer abziehbaren Schutzschicht.

Fentanylum (Fentanyl, Fentanil) ist bereits 1984 in der Anwendung mittels eines transdermalen Pflasters patentiert worden (US 45 88 580). Es hat sich seit her bestens in der Therapie von starken und/oder chronischen Schmerzzuständen, insbesondere postoperativ als auch bei Krebspatienten bewährt. Als Nebenwirkungen sind in dieser Substanzklasse der Opioide und so auch bei Fentanyl Übelkeit, Kreislaufprobleme, Verstopfung oder Pruritus und lebensbedrohliche Atemdepression zu beobachten, was eine langsame und kontinuierliche Zufuhr in den Körper erfordert. Wegen der schlechten oralen Bioverfügbarkeit von < 10% sind orale Retardformen (Retardtabletten) nicht anwendbar. Transdermal appliziert wird der first-pass- Effekt in der Leber vermieden, die Aufnahme der Substanz durch die Haut ist gut und man kann auf diese Weise lang anhaltende, gleichmäßige Blutspiegel erreichen, wenn es gelingt, eine geeignete transdermale Formulierung zu entwickeln. Aus diesen Gründen zählt die Applikation von Fentanyl aus einem transdermalen Pflaster einen stets wachsenden Marktanteil bei der Therapie starker Schmerzzustände dar.

Bei einem transdermalen System wie Durogesic ^{™} penetriert das aus der Formulierung freigesetzte Fentanyl die Hautbarriere, um durch die durchblutete Unterhaut in den systemischen Blutkreislauf zu gelangen und von dort zentral den analgetischen Effekt mittels Reaktion an den Opiatrezeptoren im Gehirn zu entfalten. Allerdings erfolgt auch aufgrund der hohen Lipophilie des Opioidanalogons eine Anreicherung im Fettgewebe, woraus wiederum eine Freigabe zu späterem Zeitpunkt in den Kreislauf erfolgen kann; man spricht hier von einem Hautdepot.

Die Penetration von Arzneistoffen durch die Haut ist weitgehend durch die physikochemischen Eigenschaften der Substanz bestimmt. Hierbei spielen im wesentlichen der Octanol/Wasser-Verteilungskoeffizient sowie die Molekülgröße eine Rolle (Potts RO, Guy RH in: Gurny R, Teubner A; Dermal and transdermal drug delivery, Wiss. Verlagsges. Stuttgart (1993)). Da der Patient es bevorzugt, ein wirksames Pflaster in einer Größe so unauffällig und klein wie möglich anzuwenden, besteht auch in diesem Falle der Wunsch, die Penetrationsrate zu steigern, wobei es eigentlich nur zwei Möglichkeiten gibt, wenn man nicht die Haut durch "Microinjektionen", Microläsionen oder das Anlegen von externen Energiequellen (z.B. lontophorese o.ä.) steigern will:
1.Erleichterung der Diffusion durch Zusatz von Penetrationsbeschleunigern oder Anwendung von elektrischer Spannung (lontophorese)
2.Steigerung der Arzneistoffkonzentration in der Grundlage auch über die Löslichkeitsgrenze hinaus (Übersättigung).

Als Penetrationsbeschleuniger werden u.a. Alkohole, Fettsäuren, Fettalkohole, einfache und mehrwertige Alkohole, Laurocapram und Tenside eingesetzt. Viele dieser Substanzen wirken jedoch über eine Störung der Barrierefunktion der Haut und sind damit als mehr oder weniger hautreizend einzustufen. Dennoch sind zahlreiche Systeme in Patentschriften beschrieben (vgl. WO 89 10 108, WO 9956782, WO 9932153 etc.).

Verträglicher ist die Verwendung von Systemen, bei denen der Wirkstoff in übersättigter Form vorliegt. Üblicherweise ist der maximale Flux einer Substanz durch die Haut durch seine Löslichkeit in der Hornhaut (Stratum corneum), welche die Hauptpenetrationsbarriere darstellt, begrenzt. Diese Sättigungskonzentration wird sich dann einstellen, wenn der Wirkstoff im Vehikel, z.B. in der Matrix des Transdermalsystems, ebenfalls in einer Konzentration vorliegt, die der Löslichkeit im Vehikel entspricht. Eine Möglichkeit, diese sog. maximale thermodynamische Aktivität weiter zu erhöhen, besteht darin, den Arzneistoff in einer die Löslichkeit im Vehikel überschreitenden Konzentration einzuarbeiten. Dies ist z.B. durch die Einarbeitung des Fentanyl in Acrylat-Copolymere möglich (WO 20024386). Die Einstellung der Übersättigung muß aber so sensibel erfolgen, daß die Übersättigungen so hoch wie möglich, aber auch so stabil wie nötig sind, da übersättigte Systeme bekanntlich metastabil sind und nach Lagerung durch Rekristallisation in den gesättigten Zustand übergehen. Das hat dann den Nachteil, daß diese Systeme aufgrund der Kristallisation zu Produktreklamationen infolge mangelnden Aspekts als auch mangelnder Klebkraft führen. Ebenfalls ist ein enger Kontakt zwischen transdermalem System und der Haut notwendig, um einen wirksamen Anteil an Fentanyl in den Zielbereich der Blutzirkulation zu erhalten.

Allerdings zählt Fentanyl wie bereits erwähnt, zu den wenigen Arzneistoffen, die aufgrund der physikochemischen Substanzeigenschaften sehr gut durch die Hautbarriere permeieren und gerne in Polymere migrieren und sich anreichern. Da die therapeutische Breite von Fentanyl gering ist und zudem auch ein Suchtpotential wie bei allen Opioiden besteht, ist ein weiterer Wunsch bei der Entwicklung eines transdermalen Fentanylpflasters der, so wenig Substanz wie möglich, aber soviel wie nötig, einzuarbeiten, damit ein therapeutischer Blutspiegel über mehrere Tage aufrechterhalten werden kann.

In der US-A 5,474,783 wird eine Polymermatrix mit einem Gemisch von zwei verschiedenen Polymeren beschrieben. Als Polymere werden hierbei ein Polyacrylat und ein Polyethylen-Vinylacetat verwendet.

Aus der DE-C-101 41 650 bzw. der WO-A-03/018075 ist ein transdermales System mit einer Deckschicht bekannt, das eine Klebermatrix mit einem Gehalt an Fentanyl sowie eine abziehbare Schutzschicht aufweist.

Die WO-A-02/074286 beschreibt ebenfalls bereits ein transdermales therapeutisches System mit einer Deckschicht, einer Klebermatrix sowie einer Schutzschicht.

Aufgabe der vorliegenden Erfindung ist es, ein verbessertes transdermales therapeutisches System der einleitend genannten Art auszubilden.

Diese Aufgabe wird erfindungsgemäß dadurch gelöst, daß eine Acrylat-Copolymer-Klebermatrix verwendet wird, die frei von Penetrationsbeschleunigern ist, wobei die Klebermatrix ein basisches Acrylat-Copolymeres ist, aus Einheiten, die ausschließlich von 2-Ethylhexylacrylat, Methylacrylat und 2-Hydroxyethylacrylat herrühren, und mit einer organischen Titan-Verbindung als Vernetzer ist.

Es wurde herausgefunden, daß die Einarbeitung des Fentanyl als Base in einen auf ganz spezielle Art vernetzten Acrylat-Copolymer sowohl eine so stabile Lösung erreicht, daß man zu einem wirksamen Produkt kommt, ohne Penetrationsbeschleuniger zusetzen zu müssen als auch eine optimale Haftung auf der Haut erhält, die der Gestalt ist, daß bei engem Kontakt zwischen dem dermalen System und der äußeren Hautbarriere über mehrere Tage bis maximal eine halbe Woche trotzdem eine Wiederentfernbarkeit jederzeit gegeben ist, ohne daß es zu Schmerzempfinden noch Hautreizungen kommt.

Es wurden mehrere Acrylatcopolymere der Firma National Starch & Chemical, BV, Zutphen, Netherland (Handelsname Durotak) getestet. So stellte sich heraus, daß ein Copolymer, der geringe Mengen an Acrylsäure enthält (Durotak 387-4350) sowie ein Graftpolymer (Durotak 87-9301 elite), der zwar keine Säure-oder Basegruppen, dafür aber ein Acryloctylamid-Graft enthält, zu reaktiv sind und zu einem deutlichen Abbau von Fentanyl innerhalb kürzester Zeit führen. Kleber ohne funktionelle Gruppen (Durotak 87-4098) erwiesen sich als ausreichend stabil, jedoch sind Kleber mit einem geringen Anteil von Hydroxyethylacrylat (Durotak 387-2510) hinsichtlich der thermodynamischen Aktivität bei gleicher Konzentration deutlich überlegen, was sich durch bessere in-vitro Permeationsraten an exzedierter Humanhaut in Franzzellen gezeigt hat.

Allerdings führt die Verwendung eines Klebers mit Hydroxyethylacrylat (Durotak 387-2510) in Gegenwart von Fentanyl zu einer Erweichung des Polymers, was zu einer zu starken Klebkraft und "kalten Fluß" der Klebermatrix führt. Beides ist unerwünscht bzw. macht ein Pflaster ungeeignet.

Es wurden mehrere Arten der Klebkrafteinstellung dieses ganz bestimmten Acrylat-Copolymeren auf Lösungsmittelbasis, wie sie von der Firma National Starch & Chemical, BV, Zutphen, Netherland unter dem Handelsnamen Durotak zur Verfügung gestellt werden, getestet. Die nachfolgende Tabelle gibt die Rezeptur-Zusammensetzungen wieder:

| Parameter | Vergleichsbeispiel 1 | Vergleichsbeispiel 2 | Vergleichsbeispiel 3 | Erfindungsgemäßes Beispiel 1 |
|---|---|---|---|---|
| Durotak 387-2510 | X | X | X | X |
| Vernetzer | - | 0.5% Aluminiumacety-Iacetonat | 5% Polybutyltitanat | 0.5% Poly-butyltitanat |
| Klebkraft in vitro [N/25 mm] | 9.1 | 6.8 | 0.6 | 3.0 |
| Klebeeigenschaften in vivo | Schmerzhaftes Entfernen incl. Hornhautschichtabrasion | Rückstände auf der Haut beim Abziehen | Klebt zu schwach | Gute Klebeeigenschaft |

Wie man ersehen kann, werden die Trageeigenschaften durch Vernetzung des basischen Durotak erreicht. Es gibt viele andere Möglichkeiten, Kohäsion und Klebeeigenschaften dieser Kleber von der Firma National Starch & Chemical (Durotak 387-2510, 387-2516) zu beeinflussen, z.B. durch Titanvernetzer, oder durch Zusatz von Feststoffen wie Aerosil oder Talkum, die in anderen Systemen durchaus zu Erfolg geführt haben (JP 2000 04447), oder durch Zusatz anderer Polymere wie Silikon, Harze, Polyisobutylenen (WO 9902141, WO 9300058), aber wenn nur der oben erwähnte Kleber Durotak 387-2510 eingesetzt wird, führt eine Anwendung von Polybutyltitanat zum besten Ergebnis, was überraschend war. Es wird scheinbar eine spezielle, unbekannte Art der Einlagerung des Wirkstoffes in die entsprechend durch Vernetzung eingestellten Kavitäten des Acrylat-Copolymers erzielt, ohne daß es zu einer Bindung oder zum irreversiblen Einschluß kommt. Das ist auch daran ersichtlich, daß bei einem Zusatz von Polybutyltitanat zu einer Formulierung mit Fentanyl, wie in der Tabelle gelistet, eine Klebkraft in-vitro von etwa 3 N/25mm resultiert, das Placebo hingegen, also die Formulierung ohne Fentanyl, Klebkraftwerte besitzt, die um den Faktor 2 höher sind (6 N/25mm).

Die Einarbeitung des Titanvernetzers bedarf einiger Fertigkeiten seitens des Fachmanns. Je nach Lieferquelle des Polybutyltitanats kann es sein, daß dieser verschieden eingearbeitet werden muß. Der Vernetzer von Aldrich (Germany) beispielsweise kann einfach nach Lösen in etwas Ethanol zu der wirkstoffhaltigen Klebermasse auf einen Schlag zugegeben werden. Verfährt man in gleicher weise mit Vernetzer von Synetix (Vertec ^{™}, UK), so entstehen im Laminat nach einigen Wochen braune Partikel. Deshalb muß man diesen Vernetzer in Heptan vorlösen, dann Ethanol zur Mischung geben (Mischungsverhältnis 60:40), so daß eine 3%ige Vernetzerlösung resultiert. Diese wird langsam unter starken Rühren der wirkstoffhaltigen Klebermasse zugegeben. Erst dann erhält man auch nach Lagerung eine einwandfreie Matrix.

Es wird dem Fachmann empfohlen, durch Vorversuche sicherzustellen, daß bei der Zugabe des Vernetzers sorgfältig vorgegangen wird, damit es nicht zu einem verstärkten Abbau von Fentanyl, insbesondere zum Auftreten der Verunreinigung D (Europäisches Arzneibuch) kommt. Dieses Produkt tritt dann bereits bei Stresslagerung von nur 1 Monat bei 40°C/75% r.F. in einer Menge von ca. 1%, bezogen auf Fentanyl, auf. Homogenisiert man zuerst den Vernetzer in der wirkstofffreien Klebermasse und gibt erst dann den gelösten Wirkstoff zu, so sollte ein Laminat frei von Verunreinigung D erhalten werden.

Darüber hinaus spielt bei den Trageeigenschaften der Träger der Matrix eine wichtige Rolle. Da das transdermale System in der stärksten Dosierung von 100 µg Fentanyl pro Stunde Abgaberate bereits eine Größe von mindestens 40 cm² erreicht, was erheblich ist, ist eine gewisse Flexibilität von Vorteil für den Tragekomfort.

Es wurden verschiedene klarsichtige Folienmaterialien getestet, die sich von der Chemie des Materials über PET (Polyester), BOPP (biaxial orientiertes Polypropylen), PE (Polethylen, Polyolefine), PU (Polyurethan) und PS (Polystyrol-Copolymer) erstreckte. Wichtig hierbei war ebenfalls, inwieweit Fentanyl ein Migrationsverhalten gegenüber den Materialien aufwies. Es zeigte sich, daß PU keine Kohäsion zur Klebermatrix erreichte und deshalb ungeeignet war. Sehr angenehme Trageeigenschaften zeigte PE, aber etwa 8-10 % des Wirkstoffes migrierten innerhalb weniger als 1 Monat bei 40°C/75% r.F. in diese Trägerfolie und standen somit nicht mehr zur Verfügung für die transdermale Absorption. Da Fentanyl als Rohstoff sehr teuer ist, wollte man nicht einen Produktionszuschlag zur Behebung einsetzen. Dieses wäre auch deshalb ungeeignet, weil die Menge an Fentanyl, die in die Folie migriert, sich über die Zeit ändert. Keine Migration wurde festgestellt in PET, gefolgt von BOPP, welche aufgrund der etwas größeren Flexibilität auch bevorzugt wird.

Als Schutzfolie wird ein dem Fachmann bekannte silikonisierte Polyesterfolie, z.B. Hostaphan RN 100 von Mitsubishi, Germany, Silikonisierung easy/easy, eingesetzt. Die Schutzfolie sollte nicht zu dünn sein (mind. 36 µm Schichtdicke, vorzugsweise 100 µm Schichtdicke), damit auch die größeren Systeme von 30 cm² und mehr noch gut durch den Patienten zu handhaben sind.

Die erfindungsgemäßen dermalen therapeutischen Systeme sind vorzugsweise so beschaffen, daß sie aus einer für den wirkstoff undurchlässigen Deckschicht, einer auf der Deckschicht haftenden wirkstoffhaltigen Kleberschicht und einer abziehbaren Schutzschicht bestehen.

Diese einfachste Form eines TDS kann in der dem Fachmann bekannten Weise hergestellt werden, indem eine Lösung des Klebers oder Klebergemisches in einem niedrigsiedenden Lösungsmittel mit dem Wirkstoff gemischt wird, die Mischung gleichmäßig auf einer abziehbaren Schutzschicht aufgetragen wird, das Lösungsmittel durch Erwärmen quantitativ entfernt und das erhaltene Produkt mit einem Träger abgedeckt wird. Die aufgebrachte wirkstoffhaltige Kleberschicht hat eine Dicke von 20 bis 500 µm.

Die nachfolgenden Ausführungsbeispiele dienen zur näheren Erläuterung der Erfindung:

### Erfindungsgemäßes Beispiel 1:

Zu 23,44 g einer 42 %igen (m/m) Lösung eines Acrylat-Klebstoffes (Durotak 387-2510, National Starch & Chemical B.V., NL-Zutphen) wird 0,056 g Polybutyltitanat langsam unter starkem Rühren in Form einer 3%igen Lösung aus Heptan : Ethylalkohol 60:40 gegeben und homogenisiert. Dazu gibt man 1,1 g Fentanyl, gelöst in 11,4 g Ethanol. Durch einstündiges Rühren wird die wirkstoffhaltige Klebermasse homogenisiert und anschließend mit einem Rakel auf einer silikonisierten, 100 µm starken Polyesterfolie (FL 2000 100 µ 1-S, Loparex B.V., NL-Apeldoorn) in einer Naßschichtdicke von 310 µm ausgestrichen. Nach der Trocknung (10 min. bei 70°C und 5 min bei 100°C) wird das klare und homogene Laminat mit einem Polyesterfilm (Hostaphan RN15, Mitsubishi, D-Frankfurt) kaschiert. Ein Pflaster der Größe 10 cm² enthält bei einem Matrixgewicht von 55,0 g/m² 5,5 mg Fentanyl.

### Beispiel 2:

Zu einer Mischung von 6,29 g einer 42 %igen (m/m) Lösung des Acrylat-Klebstoffes Durotak 387-2510 und 0,86 g einer 38,3 %igen (m/m) Lösung des Acrylat-Klebstoffes Durotak 87-4089 wird eine Lösung von 0,33 g Fentanyl in 3,7 g Ethanol gegeben. Durch einstündiges Rühren wird die Lösung homogenisiert und anschließend mit einem Rakel auf einer silikonisierten, 100 µm starken Polyesterfolie (FL 2000 100 µ 1-S, Loparex B.V., NL-Apeldoorn) in einer Naßschichtdicke von 400 µm ausgestrichen. Nach der Trocknung (15 min bei 70°C) wird das leicht trübe Laminat mit einem BOPP-Film kaschiert (Trespaphan NAA 40 µm, Trespaphan, D-Frankfurt) kaschiert. Ein Pflaster der Größe 10 cm² enthält bei einem Matrixgewicht von 55,0 g/m² 5,5 mg Fentanyl.

### Beispiel 3:

Zu einer Mischung von 4,71 g einer 42 %igen (m/m) Lösung des Acrylat-Klebstoffes Durotak 387-2510 und 2,58 g einer 38,3 %igen (m/m) Lösung des Acrylat-Klebstoffes Durotak 87-4089 wird eine Lösung von 0,33 g Fentanyl in 3,7 g Ethanol gegeben. Durch einstündiges Rühren wird die Lösung homogenisiert und anschließend mit einem Rakel auf einer silikonisierten, 100 µm starken Polyesterfolie (FL 2000 100 µ 1-S, Loparex B.V., NL-Apeldoorn) in einer Naßschichtdicke von 400 µm ausgestrichen. Nach der Trocknung (15 min bei 70°C) wird das leicht trübe Laminat mit einem BOPP-Film kaschiert (Trespaphan NAA 40 µm, Trespaphan, D-Frankfurt) kaschiert. Ein Pflaster der Größe 10 cm² enthält bei einem Matrixgewicht von 55,0 g/m² 5,5 mg Fentanyl.

### Beispiel 4:

Zu einer Mischung von 3,54 g einer 42 %igen (m/m) Lösung des Acrylat-Klebstoffes Durotak 387-2510 und 3,87 g einer 38,3 %igen (m/m) Lösung des Acrylat-Klebstoffes Durotak 87-4089 wird eine Lösung von 0,33 g Fentanyl in 3,7 g Ethanol gegeben. Durch einstündiges Rühren wird die Lösung homogenisiert und anschließend mit einem Rakel auf einer silikonisierten, 100 µm starken Polyesterfolie (FL 2000 100 µ 1-S, Loparex B.V., NL-Apeldoorn) in einer Naßschichtdicke von 400 µm ausgestrichen. Nach der Trocknung (15 min bei 70°C) wird das leicht trübe Laminat mit einem BOPP-Film kaschiert (Trespaphan NAA 20 µm, Trespaphan, D-Frankfurt) kaschiert. Ein Pflaster der Größe 10 cm² enthält bei einem Matrixgewicht von 55,0 g/m² 5,5 mg Fentanyl.

Das nachfolgenden Ausführungsbeispiel zeigt, daß ein erfindungsgemäß hergestelltes Pflaster sich in einer vergleichenden Bioverfügbarkeitsstudie an 6 gesunden Probanden im cross-over als bioequivalent zum Orginatorprodukt Durogesic erweist, wenn beide Pflasterprodukte jeweils 3 Tage getragen werden:
Die Formulierung entsprach dem erfindungsgemäßen Beispiel 1 mit der Ausnahme, daß statt mit einem Polyesterfilm (Hostaphan RN15, Mitsubishi, D-Frankfurt) mit einem BOPP-Film kaschiert (Trespaphan NAA 40 µm, Trespaphan, D-Frankfurt) wurde. Jedes getestete Pflaster der Größe 10 cm² enthielt bei einem Matrixgewicht von 55,0 g/m² 5,5 mg Fentanyl. Das Vergleichspräparat hieß Durogesic ^{™} 25 µg Membranpflaster.
Die Ergebnisse der Kinetik sind in der Tabelle zusammengefaßt:

| Name | Erfindungsgemäßes Bsp. 1 Fentanyl TDS 25 | Durogesic ^{™} 25 µg Membranpflaster |
|---|---|---|
| AUC (0-72h) | 26.723 pg/ml*h | 24.911 pg/ml*h |
| C max | 496 pg/ml | 499 pg/ml |
| T max | 33 h (9h) | 42 h |
| C peaks | 24 - 42 h | 30 - 71,8 h |
| Absorption | Etwas schneller | - |
| Abgaberate | gleich | - |
| BV (AUC) | 107,2 % (89-129,3%) | etwas geringer |
| BV (C max) | 99,5 % (80,1-123.5%) | gleich |
| Anova CV (AUC) n=6 | 15,2% | - |
| Anova CV (Cmax) n=6 | 17,7% | - |

Die Hautverträglichkeit und Nebenwirkungen erwiesen sich als vergleichbar bei beiden Produkten.

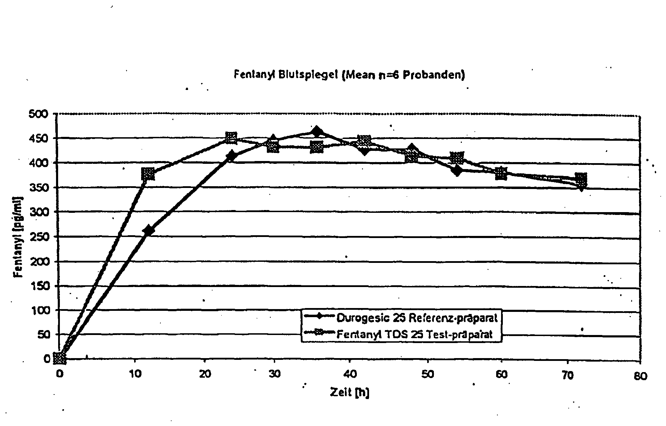

Die Graphik in Figur 1 zeigt den Verlauf der Blutspiegel beider Produkte.

Die in den Beispielen angegebenen Trockenbedingungen waren diejenigen, die im Labormaßstab zur Herstellung der Pflaster angewendet wurden. Bei Herstellung im größeren Maßstab können die Bedingungen hiervon abweichen. So wird das Produkt z.B. im Technikummaßstab in einem Trockentunnel mit 4 Trockenzonen mit einer Geschwindigkeit von 2m/min gefördert, die einzelnen Zonen weisen Temperaturen von 40 °C, 60 °C, 90 °C und 120 °C auf. Bei Herstellung im Produktionsmaßstab können wiederum andere Bedingungen herrschen, die bei den Scale up-Versuchen zu ermitteln sind.

## Patentansprüche

1. Transdermales therapeutisches System, das aus einer für den Wirkstoff undurchlässigen Deckschicht, einer auf der Deckschicht haftenden wirkstoffhaltigen Kleberschicht mit einem Gehalt an Fentanyl als Wirkstoff und einer abziehbaren Schutzschicht besteht, **gekennzeichnet durch** eine Acrylat-Copolymer-Kleberschicht, die frei von Penetrationsbeschleunigern ist, wobei die Kleberschicht basisches Acrylat-Copolymeres ist, aus Einheiten, die ausschließlich von 2-Ethylhexylacrylat, Methylacrylat und 2-Hydroxyethylacrylat herrühren, mit Polybutyltitanat in Höhe von 0,1 bis 1%, berechnet auf Molbasis, als Vernetzer und **gekennzeichnet ist durch** eine Schichtdicke der Kleberschicht im Bereich von 20 bis 500 µm.

2. Transdermales therapeutisches System nach Anspruch 1, **gekennzeichnet durch** einen Gehalt an Fentanyl in einer Konzentration von 0,1 bis 30 Gew.-%, insbesondere 5 bis 18 Gew.-%, bezogen auf das Gewicht der Klebermatrix mit Wirkstoff.

3. Transdermales therapeutisches System nach Anspruch 1 und/oder 2, **gekennzeichnet durch** einen Restgehalt an Fentanyl-Lösemittel, insbesondere Ethylalkohol, von kleiner 0,25 Gew.-%, bezogen auf das Gewicht der Klebermatrix mit Wirkstoff.

4. Transdermales therapeutisches System nach mindestens einem der vorhergehenden Ansprüche, **gekennzeichnet durch** ein Acrylat-Copolymeres als Klebermatrix, das **durch** Trocknung bei einer Temperatur von etwa 70°C oder bei einer Temperatur über 70°C gewinnbar ist.

5. Transdermales therapeutisches System nach mindestens einem der vorhergehenden Ansprüche, **gekennzeichnet durch** ein Acrylat-Copolymeres als Klebermatrix gemäß Anspruch 1, das **durch** Vernetzen der Hydroxylgruppen des Acrylat-Copolymeren und nachfolgende Zugabe des Wirkstoffs gewinnbar ist.

6. Transdermales therapeutisches System nach mindestens einem der Ansprüche 1 bis 5, **gekennzeichnet durch** einen Gehalt an Polybutyltitanat in Höhe von 0,4 bis 0,6%, berechnet auf Molbasis.

7. Transdermales therapeutisches System nach mindestens einem der vorhergehenden Ansprüche, **gekennzeichnet durch** eine Deckschicht auf Polypropylenbasis, insbesondere **durch** eine biaxial orientierte, längs- und quergerichtete Polypropylenfolie.

8. Transdermales therapeutisches System nach mindestens einem der vorhergehenden Ansprüche 1 bis 7, **gekennzeichnet durch** eine Deckschicht auf Polyesterbasis, insbesondere **durch** ein Polyestergewebe.

9. Transdermales therapeutisches System nach einem der Ansprüche 1 bis 8, **dadurch gekennzeichnet, dass** die Deckschicht als ein Matrixträger ausgebildet ist.

## Claims

1. A transdermal therapeutic system consisting of a top layer that is impermeable for the active ingredient, an active ingredient-containing adhesive layer that adheres to the top layer and has a content of fentanyl as an active ingredient, and a removable protective layer, **characterized by** an acrylate copolymer adhesive layer that is free from penetration promoters, wherein the adhesive layer is basic acrylate copolymer of units exclusively derived from 2-ethylhexyl acrylate, methyl acrylate and 2-hydroxyethyl acrylate, with polybutyl titanate from 0.1 to 1%, calculated on a molar basis, as a cross-linker and **characterized by** a layer thickness of the adhesive layer ranging from 20 to 500 µm.

2. The transdermal therapeutic system according to claim 1, **characterized by** a content of fentanyl in a concentration of 0.1 to 30% by weight, in particular 5 to 18% by weight, based on the weight of the adhesive matrix with active ingredient.

3. The transdermal therapeutic system according to claim 1 and/or 2, **characterized by** a residual content of fentanyl solvent, in particular ethyl alcohol, of less than 0.25% by weight, based on the weight of the adhesive matrix with active ingredient.

4. The transdermal therapeutic system according to at least one of the preceding claims, **characterized by** an acrylate copolymer as the adhesive matrix that can be recovered by drying at a temperature of about 70°C or at a temperature above 70°C.

5. The transdermal therapeutic system according to at least one of the preceding claims, **characterized by** an acrylate copolymer as the adhesive matrix according to claim 1 that can be recovered by cross-linking the hydroxyl groups of the acrylate copolymer and subsequent adding the active ingredient.

6. The transdermal therapeutic system according to at least one of claims 1 to 5, **characterized by** a content of polybutyl titanate from 0.4 to 0.6%, calculated on a molar basis.

7. The transdermal therapeutic system according to at least one of the preceding claims, **characterized by** a top layer on a polypropylene basis, in particular by a biaxially oriented longitudinally and transversally directed polypropylene film.

8. The transdermal therapeutic system according to at least one of the preceding claims 1 to 7, **characterized by** a top layer on a polypropylene basis, in particular by polyester fabric.

9. The transdermal therapeutic system according to one of claims 1 to 8, **characterized in that** the top layer is formed as a matrix carrier.

## Revendications

1. Système thérapeutique transdermique qui se compose d'une couche de recouvrement imperméable au principe actif, d'une couche adhésive contenant le principe actif et adhérant à la couche de recouvrement, comportant une teneur en fentanyl en tant que principe actif, et d'une couche protectrice détachable, **caractérisé par** une couche adhésive à base de copolymère d'acrylate qui est exempte d'accélérateur de pénétration, la couche adhésive étant un copolymère d'acrylate basique, composé de motifs qui proviennent exclusivement du 2-éthylhexylacrylate, du méthylacrylate et du 2-hydroxyéthylacrylate, comportant du polybutyltitanate à raison de 0,1 à 1 %, calculés sur une base en moles, en tant qu'agent de réticulation, et **caractérisé par** une épaisseur de couche de la couche adhésive dans la gamme de 20 à 500 µm.

2. Système thérapeutique transdermique selon la revendication 1, **caractérisé par** une teneur en fentanyl dans une concentration de 0,1 à 30 % en poids, en particulier de 5 à 18 % en poids, sur la base du poids de la matrice adhésive avec principe actif.

3. Système thérapeutique transdermique selon la revendication 1 et/ou 2, **caractérisé par** une teneur résiduelle en solvant de fentanyl, en particulier en alcool éthylique, inférieure à 0,25 % en poids, sur la base du poids de la matrice adhésive avec principe actif.

4. Système thérapeutique transdermique selon au moins l'une des revendications précédentes, **caractérisé par** un copolymère d'acrylate en tant que matrice adhésive, qui peut être obtenu par séchage à une température d'environ 70°C ou à une température supérieure à 70°C.

5. Système thérapeutique transdermique selon au moins l'une des revendications précédentes, **caractérisé par** un copolymère d'acrylate en tant que matrice adhésive selon la revendication 1, qui peut être obtenu par réticulation des groupes hydroxyle du copolymère d'acrylate et ajout successif du principe actif.

6. Système thérapeutique transdermique selon au moins l'une des revendications 1 à 5, **caractérisé par** une teneur en polybutyltitanate de 0,4 à 0,6 %, calculée sur une base en moles.

7. Système thérapeutique transdermique selon au moins l'une des revendications précédentes, **caractérisé par** une couche de recouvrement à base de polypropylène, en particulier par une feuille de polypropylène à orientation biaxiale, de directions longitudinale et transversale.

8. Système thérapeutique transdermique selon au moins l'une des revendications 1 à 7, **caractérisé par** une couche de recouvrement à base de polyester, en particulier par un tissu polyester.

9. Système thérapeutique transdermique selon l'une des revendications 1 à 8, **caractérisé en ce que** la couche de recouvrement est formée en tant que support de matrice.
